# FASCICULE DE BREVET EUROPEEN

(11) **EP 2 689 012 B1**
(45) Date de publication et mention de la délivrance du brevet: **03.07.2019**
(21) Numéro de dépôt: 12709345.8
(22) Date de dépôt: 21.03.2012
(51) Int. Cl.: C12N 15/00, C12P 1/00, C12Q 1/04, B01D 17/02, B01D 17/04, G01N 15/02, B03D 1/00, B04B 5/04, G01N 15/04, G01N 15/10, B01D 61/38, B01D 19/00, C12N 1/00

(54) **PROCÉDÉ DE CRIBLAGE HAUT DÉBIT DE GOUTTES PAR ÉCHANGE OSMOTIQUE ET VARIATION DE DENSITÉ**
HOCHDURCHSATZ-SCREENINGVERFAHREN VON TROPFEN DURCH OSMOTISCHEN AUSTAUSCH UND DICHTEVERÄNDERUNG
METHOD FOR HIGH-THROUGHPUT SCREENING OF DROPS BY OSMOTIC EXCHANGE AND DENSITY VARIATION

(30) Priorité: 22.03.2011 FR 1152352
(43) Date de publication de la demande: 29.01.2014
(73) Titulaire: Centre National de la Recherche Scientifique (C.N.R.S.), 75016 Paris (FR); Université Pierre et Marie Curie (Paris 6), 75005 Paris (FR); Ecole Normale Supérieure de Lyon, 69364 Lyon Cedex 07 (FR); Université Paris Diderot - Paris 7, 75205 Paris Cedex 13 (FR)
(72) Inventeur: YVERT, Gaël, F-69230 Saint Genis Laval (FR); BIBETTE, Jérôme, F-75005 Paris (FR); BAUDRY, Jean-Marie, F-75011 Paris (FR); BOITARD, Laurent, 75019 Paris (FR); BREMOND, Nicolas, F-75005 Paris (FR); COTTINET, Denis, F-75013 Paris (FR)
(74) Mandataire: Lavoix
(86) Numéro de dépôt international: PCT/EP2012/055031
(87) Numéro de publication internationale: WO 2012/126959

(56) Documents cités:
- EP-A2- 0 694 327
- WO-A2-2009/134395
- US-A- 4 649 109
- JOENSSON ET AL.: "droplet size based seperation by deterministic lateral displacement - separating droplets by cell-induced shrinking", LAB CHIP, no. 11, 14 février 2011 (2011-02-14), pages 1305-1310, XP002659831, cité dans la demande
- SHERWOOD J D ET AL: "Rates of transport through a capsule membrane to attain Donnan equilibrium", JOURNAL OF COLLOID AND INTERFACE SCIENCE, ACADEMIC PRESS,INC, US, vol. 263, no. 1, 1 July 2003 (2003-07-01), pages 202-212, XP027432840, ISSN: 0021-9797, DOI: 10.1016/S0021-9797(03)00140-1 [retrieved on 2003-07-01]

## Description

La présente invention concerne un procédé de sélection d'au moins un premier groupe de gouttes parmi une pluralité de gouttes présentes dans un milieu liquide.

Un tel procédé est destiné par exemple à effectuer un criblage de très grandes librairies de molécules d'intérêt biologique qui sont réparties dans des gouttes d'émulsion. En particulier, le procédé est destiné à sélectionner des gouttes comprenant un produit particulier parmi toutes les gouttes, ce produit pouvant résulter d'une réaction chimique ou biologique, notamment effectuée à l'aide d'un hôte cellulaire.

Pour tester en parallèle l'activité ou les propriétés d'un grand nombre de variantes de microréacteurs chimiques ou biologiques, il est connu de répartir les microréacteurs dans des gouttes d'une émulsion, puis de conduire une réaction chimique ou biologique dans chacun des microréacteurs.

Il est alors nécessaire de séparer les gouttes en fonction du produit qu'elles contiennent, notamment pour évaluer et isoler les conditions réactionnelles et les microréacteurs ayant conduit à une réaction significative.

Cette technique s'applique par exemple dans le diagnostic, ou dans l'expression de gènes. Dans ce dernier cas, il est connu de fabriquer des banques de gènes codants, par exemple pour des variantes d'une enzyme et de placer chacun des gènes obtenus dans un organisme hôte. Puis, chaque organisme hôte est disposé dans une goutte distincte en vue de déterminer si cette goutte présente une activité.

Dans un cas particulier, chaque gène est reçu dans une levure qui est placée dans une goutte particulière. La goutte contient un milieu nutritionnel et est placée dans des conditions propres à permettre une croissance biologique. Certaines levures sont alors susceptibles de croître de manière significative.

Pour isoler les gouttes dans lesquelles une croissance significative s'est produite, il est connu de placer des marqueurs fluorescents sélectifs qui sont actifs lorsque la goutte a subi une réaction significative.

Puis, les gouttes sont triées manuellement ou au moyen de machines de triage automatique pour séparer celles qui ont réagi, par exemple, par des techniques microfluidiques ou par cytométrie de flux.

De telles techniques sont relativement complexes et onéreuses. Elles permettent uniquement de discriminer des échantillons de faible taille, puisque le tri maximal pouvant être obtenu est de l'ordre de 1000 à 10000 gouttes par seconde.

Par suite, lorsque l'échantillon de départ présente une taille très importante, de telles techniques sont insuffisantes ou trop longues à mettre en oeuvre.

Le même problème de séparation se pose lorsque ces techniques de criblage sont utilisées dans le domaine du diagnostic, dans le domaine de la détection de molécules très diluées ou encore lorsqu'un grand nombre de molécules test doivent être testées simultanément avec un même réactif.

Comme précédemment, il est nécessaire de séparer les gouttes à grand débit, faute de quoi le test est trop fastidieux à mettre en oeuvre.

Pour pallier ces problèmes, on connait de l'article « Droplet Size Based Séparation by Deterministic Lateral Displacement » publié dans la revue « Lab on a Chip » le 14 février 2011, un procédé du type précité dans lequel le volume de certaines gouttes à séparer est modifié par flux osmotique. La variation de volume des gouttes est faible.

Les gouttes sont ensuite séparées suivant leur volume en les faisant s'écouler horizontalement à travers un support muni de picots. La densité des gouttes n'intervient pas dans la séparation et reste sensiblement constante. Néanmoins, cette méthode de séparation est fastidieuse à mettre en oeuvre et n'est pas très efficace.

US 4,646,109 décrit un procédé du type précité.

Un but de l'invention est donc de disposer d'un procédé de séparation d'un groupe de gouttes particulier parmi un grand nombre de gouttes, qui soit simple et rapide à mettre en oeuvre, tout en permettant une récupération rapide des gouttes criblées.

Un autre but de l'invention est de disposer d'un procédé qui permet de cribler, à grand débit et de manière simple à mettre en oeuvre, des propriétés difficilement accessibles aujourd'hui, comme le métabolisme d'un microorganisme.

A cet effet, l'invention a pour objet un procédé selon la revendication 1.

Le procédé selon l'invention peut comprendre l'une ou plusieurs des caractéristiques des revendications 2 à 13 ou des caractéristiques suivantes, prises isolément ou suivant toute combinaison techniquement possible :
- les gouttes à séparer présentent initialement une densité relativement monodisperse, la variation de densité des gouttes du premier groupe de gouttes après l'étape de flux osmotique étant supérieure à la polydispersité initiale des densités des gouttes à séparer ;
- la variation de densité des gouttes du deuxième groupe de gouttes après l'étape de flux osmotique est inférieure à la polydispersité initiale des densités des gouttes à séparer.

L'invention a également pour objet un procédé selon la revendication 14.

Le procédé selon l'invention peut comprendre l'une ou plusieurs des caractéristiques des revendications 15 à 25 ou des caractéristiques suivantes, prises isolément ou suivant toute combinaison techniquement possible :
- les gouttes à séparer présentent initialement une densité relativement monodisperse, la variation de densité des gouttes du premier groupe de gouttes après l'étape de flux osmotique étant supérieure à la polydispersité initiale des densités des gouttes à séparer ;
- la variation de densité des gouttes du deuxième groupe de gouttes après l'étape de flux osmotique est inférieure à la polydispersité initiale des densités des gouttes à séparer.

L'invention sera mieux comprise à la lecture de la description qui va suivre, donnée uniquement à titre d'exemple, et faite en se référant aux dessins annexés, sur lesquels :
- la figure 1 est un schéma synoptique fonctionnel d'un premier procédé selon l'invention ;
- la figure 2 est une vue d'une pluralité de gouttes à séparer par un premier procédé selon l'invention, au début de la mise en oeuvre du procédé ;
- la figure 3 est une vue analogue à la figure 2, après l'étape de variation du volume d'au moins un premier groupe de gouttes ;
- la figure 4 est une courbe de la variation relative du volume d'un premier groupe de gouttes et d'un deuxième groupe de gouttes, lors de la mise en oeuvre du procédé selon l'invention ;
- la figure 5 est une vue schématique illustrant un premier exemple d'application du procédé selon l'invention ;
- la figure 6 est une vue schématique analogue à la figure 5 illustrant un deuxième exemple d'application du procédé selon l'invention ;
- la figure 7 est une vue schématique d'une première mise en oeuvre de l'étape de séparation des gouttes suivant leur densité dans le procédé selon l'invention ;
- la figure 8 est une vue analogue à la figure 7, après la séparation ;
- la figure 9 illustre la variation de la densité en fonction du volume des gouttes lors d'une étape de séparation spontanée des gouttes d'un premier groupe de gouttes et d'un deuxième groupe de gouttes ;
- la figure 10 est une vue illustrant la vitesse de sédimentation ou de crémage d'une goutte au fur et à mesure de sa réduction de volume (densification) dans un milieu liquide donné ;
- la figure 11 est une courbe représentant la densité en fonction de la température pour illustrer une méthode de séparation du premier groupe de gouttes par variation de température ;
- la figure 12 est une vue schématique en coupe partielle d'une variante d'étape de séparation des gouttes par le procédé selon l'invention ;
- la figure 13 est une vue analogue à la figure 12 d'une autre variante d'étape de séparation des gouttes par le procédé selon l'invention ;
- la figure 14 est une vue analogue à la figure 2 pour une deuxième procédé selon l'invention ; et
- la figure 15 est une vue analogue à la figure 3 pour le deuxième procédé selon l'invention.

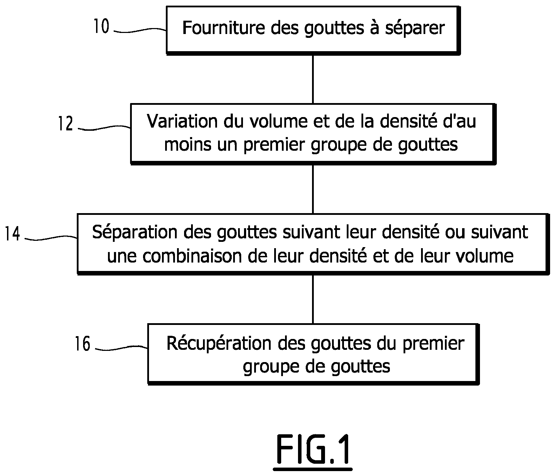

Les étapes principales d'un premier exemple de procédé selon l'invention sont représentées schématiquement sur la figure 1.

Ce procédé comprend une étape 10 de fourniture d'une multitude de gouttes à séparer, une étape 12 de variation du volume d'au moins un premier groupe de gouttes à séparer parmi les gouttes à séparer, puis une étape 14 de séparation des gouttes suivant leur densité ou une combinaison avec le volume.

Le procédé comprend éventuellement une étape 16 de récupération des gouttes du premier groupe de gouttes.

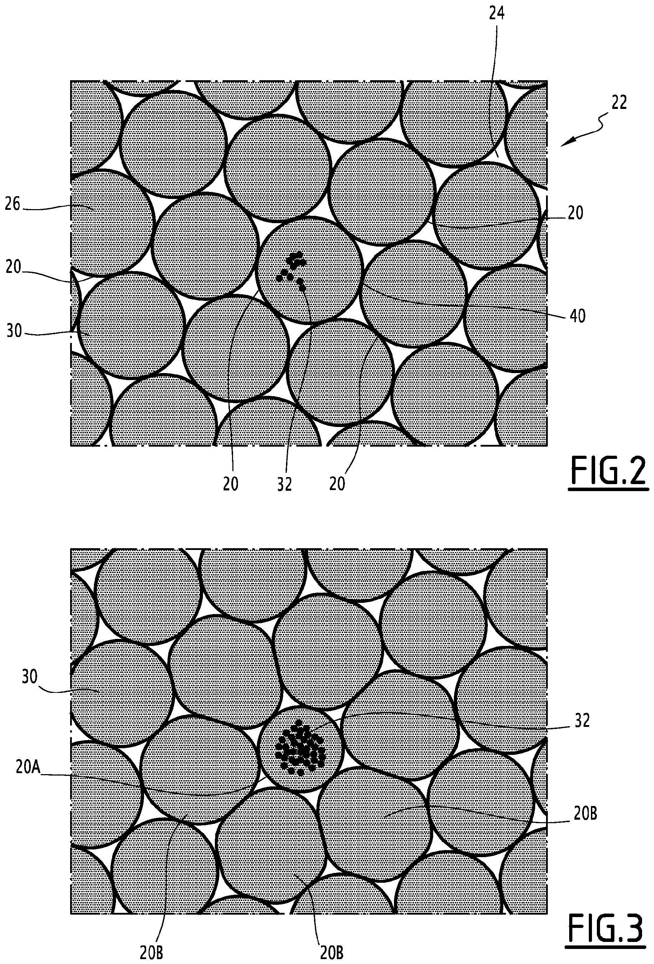

Comme illustré par la figure 2, les gouttes 20 à séparer sont initialement fournies sous forme d'une émulsion 22 tridimensionnelle de gouttes dispersées dans un milieu continu 24.

L'émulsion est par exemple une émulsion de gouttes 20 d'eau dispersées dans une phase continue 24 huileuse. En variante, les gouttes 20 sont des gouttes de phase huileuse/organique dispersées dans une phase continue 24 aqueuse. En tout état de cause, les deux fluides formant respectivement la phase continue 24 et les gouttes 20 sont sensiblement immiscibles.

L'émulsion 22 est une émulsion concentrée de gouttes. Par « émulsion concentrée », on entend que la phase dispersée constituant les gouttes 20 représentée plus de 60 % en volume du volume de l'émulsion 22.

La taille des gouttes est par exemple comprise entre 5µm et 500µm. Le nombre de gouttes 20 dans l'émulsion 22 est supérieur à 10¹⁰, et est notamment supérieur à 10¹².

Les gouttes 20 fournies initialement sont relativement monodisperses.

Par « relativement monodisperses », on entend que la polydispersité des gouttes telle que mesurée par diffraction aux petits angles ou par des mesures optiques est inférieure à 15 %.

Cette monodispersité est mesurée par exemple par la méthode de mesure optique suivante.

Les gouttes sont placées dans une chambre de 1 cm² et de 40 µm d'épaisseur. Des images de l'émulsion sont prises à l'aide d'une caméra et analysées pour mesurer la taille de chaque gouttes permettant ainsi d'avoir accès à la distribution de taille.

Les gouttes 20 comprennent toutes au moins une phase liquide commune 30 à toutes les gouttes. Certaines gouttes 20 comportent au moins un composé 32 initialement différent pour chaque goutte 20.

L'émulsion 22 est par exemple réalisée par des techniques microfluidiques comprenant par exemple des puces microfluidiques multiplexées ou encore par des techniques d'émulsification en masse à travers des membranes.

Dans un mode de réalisation avantageux, chaque goutte 20 constitue un microréacteur chimique ou biologique. Ainsi, chaque goutte 20 contient initialement au moins un réactif de départ destiné à réagir, et au moins un agent réactionnel, destiné à faire réagir le réactif de départ pour le transformer en au moins un produit final.

La goutte 20 comprend en outre éventuellement au moins un agent susceptible de faire varier les conditions de la réaction du produit de départ avec l'agent réactionnel.

Dans un premier exemple, le réactif de départ varie de goutte à goutte, de sorte que différents réactifs de départ sont soumis au même agent réactionnel pour déterminer si le réactif de départ est susceptible de réagir avec l'agent réactionnel afin d'obtenir du produit final.

Dans ce cas, les réactifs de départ sont par exemple des composés chimiques différents susceptibles de réagir avec l'agent réactionnel.

En variante, le réactif de départ est commun aux différentes gouttes 20 et les gouttes 20 comprennent des agents réactionnels différents pour déterminer si chaque agent réactionnel est susceptible de réagir avec le même réactif de départ pour former un produit final.

Dans ce cas, les agents réactionnels sont par exemple des catalyseurs homogènes ou hétérogènes, de nature différente selon les gouttes 20, pour permettre de tester l'activité de ces catalyseurs vis-à-vis du même réactif de départ.

Dans une variante, l'agent réactionnel est par exemple un microorganisme, tel qu'une bactérie ou une levure ou encore une cellules isolée d'organisme supérieur. Les différentes cellules présentent dans les différentes gouttes 20 présentent des caractéristiques distinctes, par exemple des gènes distincts.

Dans encore une autre variante, chaque goutte contient le même réactif de départ, le même agent réactionnel, et au moins un agent additionnel différent.

Dans encore une autre variante, seules certaines gouttes 20 contiennent un réactif de départ susceptible de réagir avec un agent réactionnel donné.

Dans ce cas, le procédé selon l'invention permet d'isoler des produits qui peuvent être très dilués dans un milieu continu donné.

Dans la première étape du procédé, l'émulsion concentrée 22 est par exemple formée par des techniques d'émulsification classiques ou par des techniques microfluidiques.

Les gouttes 20 obtenues sont relativement monodisperses comme illustré par la figure 2. Par suite, les gouttes présentent une densité relativement monodisperse.

Puis, à l'étape 12 de variation du volume, chaque goutte 20 à séparer est placée au contact d'une interface 40 en vue de réaliser un échange osmotique avec un milieu continu situé à l'opposé de l'interface 40 qui va servir de « référence osmotique ».

Dans le mode de réalisation des figures 2 et 3, chaque goutte 20 à séparer est avantageusement placée au contact d'au moins une goutte 20 adjacente.

Avantageusement, chaque goutte 20 est placée au contact d'une pluralité de gouttes 20 adjacentes situées tout autour de la goutte 20. En particulier, le contact est tridimensionnel. Ainsi, la goutte 20 est placée au contact de gouttes 20 sensiblement dans le même plan que la goutte 20, et aussi avec au moins une goutte 20 située dans un plan sécant avec le plan précité.

Du fait de leur contenu, au moins certaines gouttes 20A présentent un potentiel chimique différent des gouttes 20B adjacentes.

A contrario, aucune réaction significative ne s'étant produite dans les gouttes 20B, leur potentiel chimique du solvant reste sensiblement constant. Les gouttes 20B contiennent par exemple exclusivement du solvant, ou contiennent un réactif n'ayant pas réagi avec l'agent réactionnel.

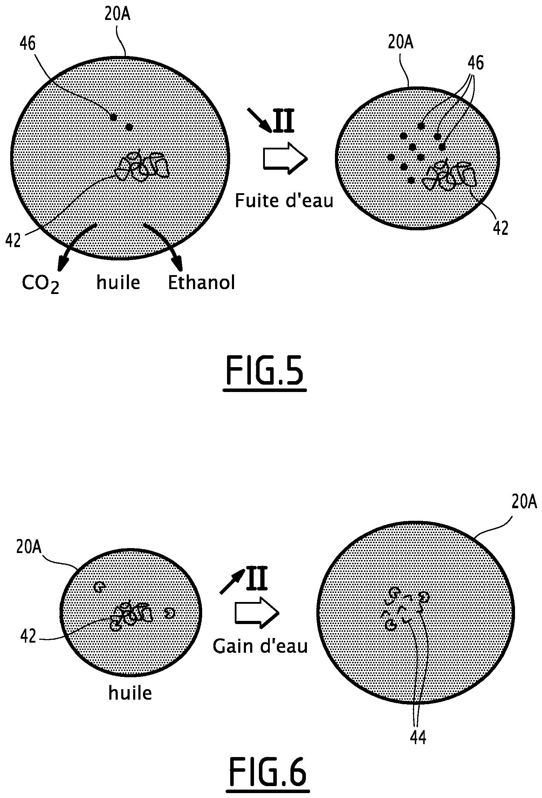

En particulier, dans l'exemple illustré par la figure 5, lorsque les gouttes 20A sont des microréacteurs, la réaction du réactif de départ contenu dans au moins une goutte 20A peut engendrer un produit final qui est au moins partiellement soluble dans la phase continue 24.

Le produit final quitte alors la goutte 20A, ce qui entraîne un flux de phase liquide 30 hors de la goutte 20A pour rééquilibrer les potentiels chimiques avec les gouttes adjacentes 20B.

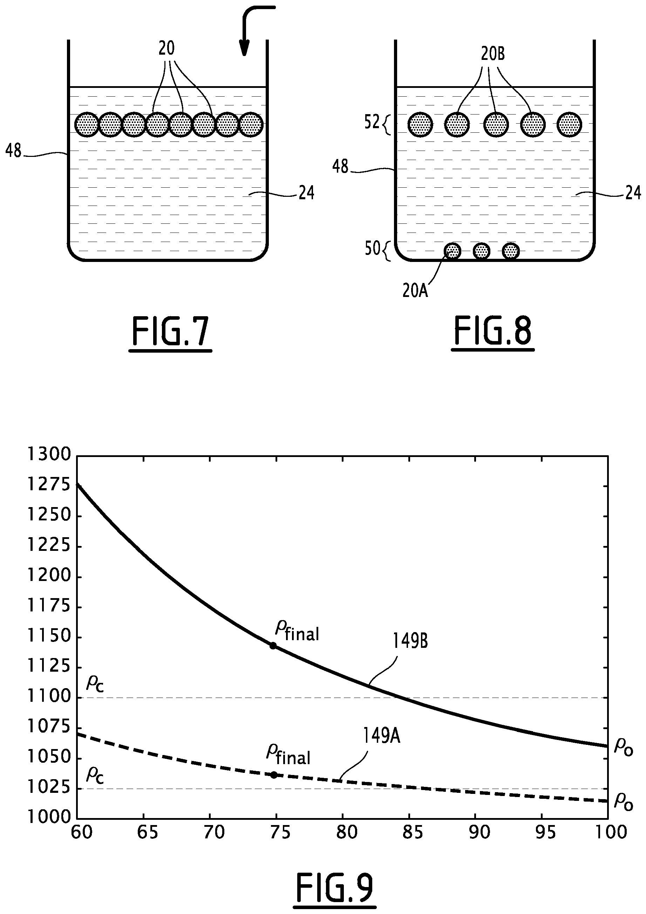

Dans ce cas, comme représenté schématiquement sur la figure 5, la taille de chaque goutte 20A d'un premier groupe de gouttes diminue du fait de la forte consommation d'un des réactifs contenus initialement dans la goutte 20A par les agents réactionnels 46 présents dans la goutte 20A, ce qui se traduit comme représenté sur la figure 9 pour une augmentation de la densité d'autant plus importantes que les gouttes contiennent un polymère non soluble dans la phase continue.

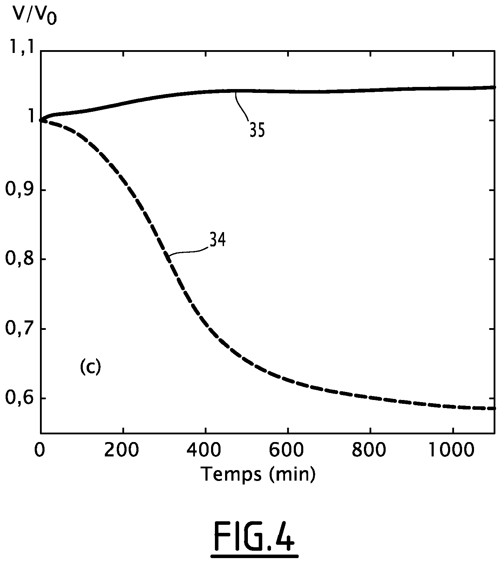

Le potentiel chimique du solvant dans la goutte 20A diminue donc, provoquant une fuite du solvant vers les gouttes adjacentes 20B et une diminution correspondante du volume de chaque goutte du 20A du premier groupe de gouttes, comme illustré par la courbe 34 sur la figure 4.

Comme illustré par la courbe 35 sur la figure 4, le volume de chaque goutte adjacente 20B augmente légèrement, mais de manière moins importante en proportion que la diminution du volume des gouttes 20A et selon le ratio de gouttes 20A et 20B.

Alternativement, dans le cas illustré par la figure 6 où le nombre d'espèces 44 présentes dans la goutte 20A augmente du fait de la réaction s'y produisant, le potentiel chimique du solvant dans la goutte 20A augmente de manière associée. La goutte 20A augmente alors de volume en captant par échange osmotique à travers l'interface 40 de la phase liquide 30 présente dans les gouttes adjacentes 20B, ce qui rééquilibre les potentiels chimiques et se traduit par une diminution de la densité des gouttes 20A.

Ainsi, dans ce cas, le volume des gouttes 20A augmente et le volume des gouttes 20B adjacentes, dans lesquelles aucune action ne s'est produite, diminue de manière correspondante.

L'étape de variation du volume comprend donc la variation du volume d'au moins un premier groupe de gouttes 20A dans lequel une variation de potentiel chimique s'est produite pour faire augmenter ou diminuer le volume de ces gouttes 20A, et la variation inverse du volume des gouttes adjacentes 20B pour lesquelles aucune variation significative de potentiel chimique ne s'est produite.

Ainsi, la variation de volume (diminution ou augmentation) des gouttes 20A du premier groupe de gouttes est supérieure à la polydispersité. En particulier, cette variation est supérieure à 20% et est notamment supérieure à 30%, voire supérieure à 40 %, comme illustré par la figure 4.

Compte tenu de l'effet de dilution entre les différentes gouttes 20B adjacentes à la goutte 20A, la variation de volume de chaque goutte 20B est inférieure à 40 %, notamment inférieure à 10 %. En outre, un grand nombre de gouttes 20 restent de volume semblablement constant. Dans une variante, la variation de volume des gouttes 20B est supérieure à 40%, mais reste dans le sens opposé de la variation de volume des gouttes 20A.

Selon l'invention, la variation de volume des gouttes 20A conduit à une variation significative de la densité des gouttes 20A. Ainsi, la variation significative de densité entre la densité initiale de chaque goutte du premier groupe de gouttes 20A avant échange osmotique et la densité finale de chaque goutte du premier groupe de gouttes 20A après échange osmotique est supérieure à 12% notamment à 25%.

La variation de densité est d'autant plus importante que la masse initiale contenue dans chaque goutte 20 est plus importante.

Dans le cas où les gouttes 20 sont des microréacteurs, cette variation permet d'identifier les microréacteurs dans lesquels une réaction significative s'est produite, ce qui a produit une variation significative de la densité.

Comme on l'a vu plus haut, la variation de potentiel chimique résulte soit de la consommation du réactif de départ pour former un produit final qui est soluble dans la phase continue 24 et qui quitte la goutte 20A, soit de l'obtention d'un nombre d'espèces supérieur dans la goutte 20A, ou plus généralement d'autres phénomènes physico-chimiques susceptibles d'affecter le potentiel chimique.

Dans le cas où la goutte 20 contient un microorganisme 46 (voir figure 5), le réactif de départ peut être un milieu susceptible d'être consommé par le microorganisme pour assurer son développement, le microorganisme produisant des composés solubles dans la phase continue 24.

En particulier, le produit de départ est avantageusement un sucre, qui est consommé par le microorganisme pour assurer sa croissance. La consommation du sucre forme du dioxyde de carbone et au moins un alcool qui diffusent dans la phase continue, provoquant une diminution du potentiel chimique de la goutte 20A.

Alternativement, lorsque le produit de départ 42 se décompose en une pluralité d'espèces 44, notamment dans le cadre de la réaction de lyse de protéines, les résidus 44 restant dans la goutte 20A, le potentiel chimique du solvant dans la goutte 20A augmente, ce qui engendre une augmentation correspondante de volume et par conséquent une diminution de la densité.

A l'étape de séparation 14, les gouttes 20A du premier groupe de gouttes, qui ont subi une variation significative de densité, sont séparées des autres gouttes 20B. Ainsi, seules les gouttes ayant subi une variation significative de leur volume et donc de leur densité vont être séparées.

De préférence, cette séparation se fait de manière spontanée, en particulier sous l'effet de la gravité.

Selon l'invention, les gouttes 20A, 20B sont séparées suivant leur densité qui est directement corrélée à leur variation de volume.

A cet effet, les gouttes 20 sont avantageusement chargées avec un composé non soluble dans la phase continue, tel qu'un polymère 42 (visible sur la figure 5) de masse molaire suffisamment élevée pour ne pas affecter la pression osmotique. La masse molaire du polymère 42 est par exemple supérieure à 10000g/mol. Le polymère représente avantageusement plus de 5 % en masse de la goutte.

Dans ce cas, et comme illustré sur la courbe 149B de la figure 9, la densité de la goutte 20 varie significativement lors de sa diminution ou augmentation de taille, notamment de plus de 40%.

L'effet du composé non soluble sur la variation de densité est illustré par la Figure 9 qui montre la variation 149A de densité observée en l'absence de composé non soluble et la même variation 149B en présence du composé non soluble.

Les gouttes 20A qui subissent une diminution de taille ont une densité qui augmente progressivement, de sorte que leur flottabilité dans la phase continue diminue.

Les gouttes 20A vont donc se répartir dans la phase continue en fonction de leur densité propre par rapport à la densité de la phase continue.

Dans une méthode illustrée par les figures 7 et 8, la variation de volume des gouttes 20A est associée à une variation de densité entre une densité initiale po illustrée sur la figure 9 et une densité ρ_{f} finale. Dans ce cas, la densité ρ_{c} de la phase continue 24 est choisie pour être comprise entre la densité initiale ρₒ des gouttes 20 et la densité finale ρ_{f} des gouttes 20A du premier groupe de gouttes.

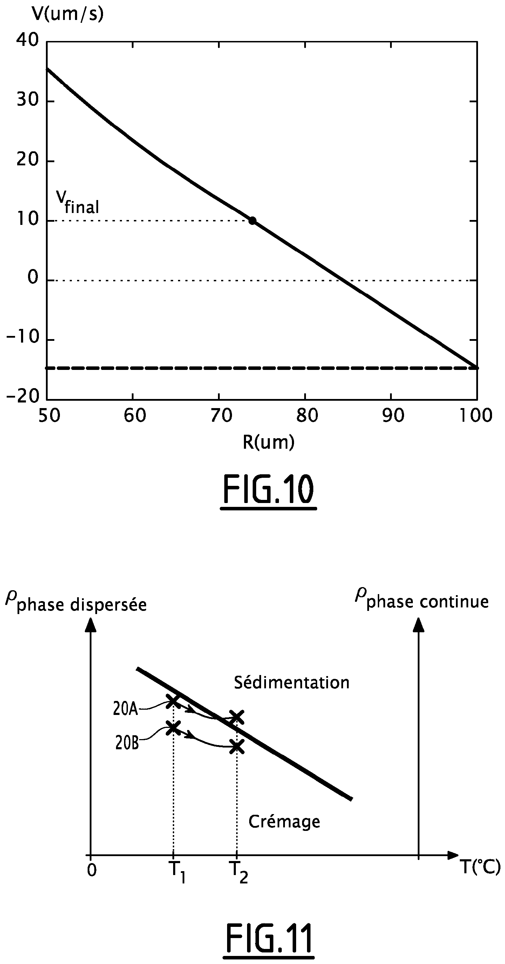

La densité des gouttes 20B du deuxième groupe de gouttes restant sensiblement constante, du fait de la faible variation de volume de ces gouttes, il est possible de séparer facilement et spontanément les gouttes 20A du premier groupe de gouttes des autres gouttes 20B. Ceci est illustre par la vitesse de sédimentation v_{final} illustrée sur la figure 10.

Dans l'exemple illustré par les figures 7 et 8, les gouttes 20B présentent une densité initiale sensiblement égale ou inférieure à la densité de la phase continue.

Les gouttes 20A du premier groupe de gouttes voient leur volume diminuer à l'étape 12. Leur densité augmente donc et passe au-dessus de la densité de la phase continue.

Les gouttes 20A du premier groupe de gouttes subissent alors une sédimentation et peuvent être récupérées sous forme d'une couche 50, comme cela est illustré par la figure 8. Une couche 52 de gouttes 20B reste en suspension.

En variante, un additif est ajouté dans la phase continue 24, par exemple à la fin de la réaction pour modifier la densité ρ_{c} de la phase continue 24 et ainsi placer cette densité ρ_{c} entre la densité des gouttes 20A du premier groupe de gouttes et la densité des gouttes 20B du deuxième groupe de gouttes.

Dans tous les cas, la séparation des gouttes 20A, 20B peut être accélérée par centrifugation.

Dans une autre variante, la température de l'émulsion 22 est modifiée après la variation de volume des gouttes 20A du premier groupe de gouttes. La densité de la phase continue 24, la densité des gouttes 20A du premier groupe de gouttes et la densité des gouttes 20B du deuxième groupe de gouttes varient de manière différentielle en fonction de la température (figure 11), ce qui provoque la séparation spontanée des gouttes 20A, 20B en fonction de leur densité (comme pour la figure 8).

Comme décrit précédemment, différentes couches 50, 52 (figure 8) contenant respectivement les gouttes 20A du premier groupe de gouttes et les gouttes 20B du deuxième groupe de gouttes sont alors formées.

Une fois les gouttes 20A du premier groupe de gouttes séparées des gouttes 20B du deuxième groupe de gouttes, elles sont récupérées à l'étape 16. Ceci permet d'isoler facilement et rapidement les gouttes 20A qui ont subi une variation de potentiel chimique importante, parmi un très grand nombre de gouttes initiales.

Le contenu des gouttes 20A est alors récupéré et/ou analysé, si nécessaire.

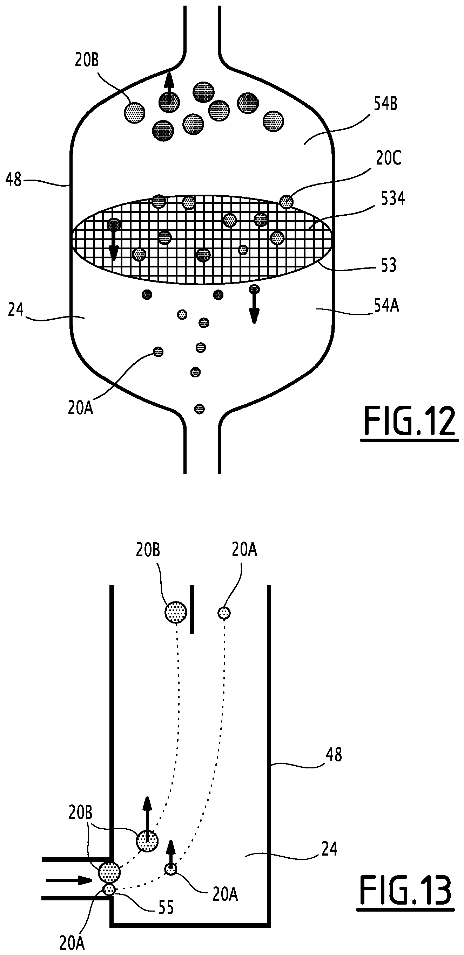

Dans une variante, représentée sur la Figure 12, les gouttes 20A, 20B obtenues après variation de volume sont placées dans une phase continue 24 de densité supérieure à celle des gouttes 20B et inférieure aux gouttes 20A.

La phase continue 24 est reçue dans un récipient 48 muni d'un filtre intermédiaire 53 plongé dans la phase continue 24. Le filtre 53 définit des ouvertures de passage 53A de dimension maximale inférieure à une dimension critique des gouttes 20A.

Les gouttes 20A, 20B sont alors placées dans la région supérieure 54B du récipient située au dessus du filtre 53. Les gouttes 20B remontent spontanément sous l'effet de la gravité. Les gouttes 20A sédimentent vers le filtre 53. Toutefois, seules les gouttes 20A ayant subi une réduction significative de volume passent à travers les ouvertures 53A du filtre 53 pour atteindre la région inférieure 54B du récipient 48 située au dessous du filtre 53. Les gouttes 20A sont alors récupérées, comme décrit plus haut. Dans une variante, un groupe de gouttes 20C ayant subi une variation insuffisante de volume reste retenu sur le filtre.

A titre d'exemple, un filtre de 50µm peut être utilisé pour séparer des gouttes d'une émulsion de gouttes 20 de 70µm de diamètre dont les gouttes 20A subissent une réduction de volume de 90%.

Dans une autre variante, illustrée figure 13, les gouttes 20A, 20B sont injectées horizontalement au voisinage du fond du récipient 48 à une vitesse horizontale donnée. Le récipient 48 contient une phase continue 24 de densité supérieure à celle des gouttes 20A, 20B.

Sous l'effet du champ gravitationnel, les gouttes 20A, 20B sont déviées vers le haut et leur trajectoire se courbe. La déviation horizontale des gouttes sera cependant dépendante du rayon de la goutte 20A, 20B. La séparation est engendrée par la combinaison entre la variation de densité et de volume des gouttes 20A, par rapport aux gouttes 20B.

Ainsi, les gouttes 20A de tailles relativement plus petites seront déviées en étant relativement plus éloignées horizontalement de l'ouverture 55 d'injection des gouttes, alors que les gouttes 20B de tailles plus grandes seront déviées en étant relativement plus proches horizontalement de l'ouverture 55.

Le récipient 48 présente avantageusement une forme de révolution autour d'un axe A-A'.

En variante, lorsque la densité de la phase continue est choisie à une valeur intermédiaire entre les gouttes 20A et les gouttes 20B, seules les gouttes 20B sont déviées vers le haut.

Les gouttes 20A ayant subi une diminution de volume sont déviées vers le bas.

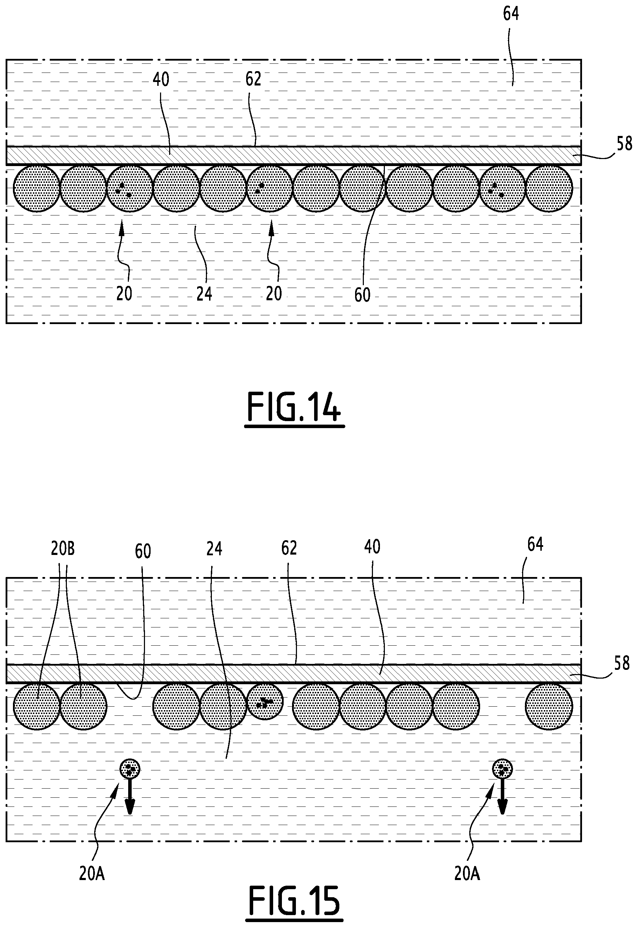

Dans un deuxième procédé selon l'invention, représenté sur les figures 14 et 15, l'interface 40 est formée au moins partiellement par une membrane 58 semi-perméable permettant la diffusion de composés chimiques de faible masse molaire (inférieure à 180g/mol) et du solvant, mais empêchant le passage des gouttes 20.

Une monocouche de gouttes 20 à séparer est déposée sous la surface 60 de la membrane 58. Une surface opposée 62 de la membrane est mise en contact avec un fluide 64 de même nature et de même potentiel chimique que le liquide initialement contenu dans les gouttes 20.

Lors de la variation de potentiel chimique des gouttes 20A du premier groupe de gouttes, une partie de la phase liquide 30 contenue dans les gouttes 20A migre à travers l'interface 40 vers le fluide 64, ce qui provoque une augmentation de la densité des gouttes 20A qui vont sédimenter.

Dans une variante (non représentée), la variation de potentiel chimique dans les gouttes 20A provoque une migration de phase liquide 30 contenue dans le volume 64 à travers la membrane 60 pour faire gonfler les gouttes 20A du premier groupe de gouttes.

Puis les gouttes 20A du premier groupe de gouttes sont séparées des autres gouttes 20B dans une étape d'auto-séparation 14 analogue à celle décrite précédemment.

Les gouttes 20A ayant subi une variation significative de densité sont alors récupérées. Le contenu des gouttes 20A est alors récupéré et/ou analysé, si nécessaire.

Dans une autre variante, la variation de volume des gouttes 20 est réalisée de sorte qu'une pluralité de classes de gouttes 20A ayant subi une variation significative de densité après la mise en oeuvre du flux osmotique sont obtenues. Les différentes classes de gouttes 20A obtenues, par exemple au moins deux ou trois classes, sont séparées suivant leur densité.

Des exemples non limitatifs de mise en oeuvre de l'invention vont maintenant être décrits.

### Exemple 1 : Criblage d'une pluralité de levures

Une pluralité de levures présentant des caractéristiques génétiques différentes sont initialement disposées dans une phase aqueuse contenant un milieu de culture tel que du glucose. Une émulsion de la phase aqueuse dans une huile est formée pour former une pluralité de gouttes 20, chaque goutte 20 contenant au plus une levure.

Les différentes gouttes 20 contiennent chacune des levures de caractéristiques différentes.

L'émulsion 22 est concentrée de sorte que chaque goutte 20 est mise au contact d'une pluralité de gouttes adjacentes 20. Les gouttes formées 20 sont monodisperses.

Dans certaines gouttes 20A d'un premier groupe de gouttes, une croissance des levures est observée, alors que dans les autres gouttes 20B, aucune croissance n'est observée ou elles sont vides.

La croissance des levures engendre la multiplication de microorganismes dans les gouttes 20A du premier groupe de gouttes. Une consommation accrue de glucose se produit dans les gouttes 20A, ce qui forme du dioxyde de carbone et de l'éthanol. L'éthanol et le dioxyde de carbone migrent dans la phase continue 24.

Un échange osmotique se produit alors entre les gouttes 20B adjacentes aux gouttes 20A pour rétablir l'équilibre de potentiel chimique entre les gouttes 20A, 20B.

A cet effet, de la phase liquide 30 présente dans les gouttes 20A traverse l'interface 40 entre les gouttes 20A, 20B et diffuse dans les gouttes adjacentes.

Le volume des gouttes 20A diminue significativement, par exemple de plus de 40 %, notamment de plus de 90 %. Ceci se traduit pour des gouttes contenant initialement au moins 6% en masse de polymère, par une augmentation de la densité de 12% notamment plus de 25%. Le volume des gouttes adjacentes augmente de moins de 10 %, notamment de moins de 2 % avec une faible variation de densité, négligeable par rapport à celle des gouttes 204.

Puis, les gouttes 20A sont séparées des autres gouttes 20B par l'une des méthodes décrites plus haut, et sont récupérées.

### Exemple 2 : Détection d'un agent pathogène très dilué dans une phase continue

Une solution dans laquelle on désir tester la présence d'agent pathogène, est diluée dans du milieu qui favorise la croissance des pathogènes. Le mélange est ensuite fragmenté en une émulsion. Les gouttes contenant les agents pathogènes vont subir une réduction de taille. Connaissant le volume initial, le volume des gouttes et le nombre de gouttes qui se sont densifiées, on peut alors avoir accès au taux de contamination de l'échantillon à tester, récupérer les agents pathogènes pour les identifier)

### Exemple 3 : Criblage de la réaction d'une banque de molécules chimiques par un même réactif : recherche d'inhibiteurs pour une enzyme de lyse (type protéase).

Une librairie de gouttes contenant chacune un inhibiteur potentiel est fusionnée avec des gouttes contenant les réactifs (enzyme + polymère ou protéine). Les gouttes contenant des molécules chimiques non inhibitrices vont gonfler du fait de l'augmentation du potentiel chimique associée à la lyse protéique (augmentation du nombre d'objets dans la goutte). Les gouttes contenant les inhibiteurs vont légèrement diminuer. La séparation des deux types de gouttes va permettre de collecter les inhibiteurs.

### Exemple 4 : Criblage d'une série de catalyseurs hétérogènes ou homogènes : évolution dirigée sur des enzymes.

Une bibliothèque de mutants de l'enzyme que l'on souhaite améliorer est constituée par les méthodes classiques de mutagenèse. On utilise la méthode de compartimentalisation in vitro développée par Tawfik et collaborateurs pour encapsuler dans chaque goutte un gène unique. Chaque goutte contient un gène muté, les enzymes de transcription pour pouvoir synthétiser l'enzyme ainsi que les réactifs sur lesquels doit agir l'enzyme. Soit l'enzyme a une activité de lyse auquel cas les gouttes d'intérêt auront gonflé. Soit elle a une activité de polymérase auquel cas le nombre d'espèces dans la goutte diminue, ce qui se traduit par des gouttes qui diminuent de volume (densification).

Comme indiqué plus haut, le procédé selon l'invention est mis en oeuvre à l'aide de gouttes 20 sensiblement immiscibles avec la phase continue 24.

En particulier, la solubilité du fluide formant les gouttes 20 dans le fluide formant la phase continue 24 est inférieure à 0,1 % en masse, à 25°C.

De même, le composant non soluble dans la phase continue présente une solubilité avantageusement inférieure à 0,01 % en masse dans la phase continue, telle que mesurée à 25°C.

Le procédé de séparation selon l'invention permet donc de séparer de manière très efficace des gouttes qui sont « actives » par rapport à des gouttes qui ne sont pas « actives ».

L'activité des gouttes se traduit par une variation significative de densité qui est avantageusement amplifiée par la présence du composant non soluble dans la phase continue dans les gouttes 20, prise en combinaison avec une variation de volume associée à l'activité éventuelle de la goutte 20.

Cette activité peut être une activité de croissance de micro-organismes mais aussi une activité métabolique, ou enzymatique. Ainsi, le procédé selon l'invention permet non seulement de séparer des gouttes dans lesquelles un nombre de micro-organismes (par exemple des cellules ou des levures) a augmenté par rapport à des gouttes ne comportant pas de micro-organismes ou comportant moins de micro-organismes que les gouttes ayant fait l'objet d'une croissance.

Le procédé selon l'invention s'applique également à des gouttes qui conservent un nombre de micro-organismes constant, mais qui présentent des activités métaboliques et/ou enzymatiques différentes. Le procédé selon l'invention ne s'applique donc pas simplement à la division de cellules, mais peut s'appliquer à des réactions enzymatiques ou encore de PCR. Ceci permet de détecter des activités se traduisant par une augmentation de la taille de la goutte et une diminution de la densité.

## Revendications

1. Procédé de sélection d'au moins un premier groupe de gouttes (20A) parmi une pluralité de gouttes (20) présentes dans une phase continue (24), les deux fluides formant respectivement la phase continue et les gouttes étant immiscibles, le procédé comprenant les étapes suivantes :
- mise en contact de gouttes (20) à séparer avec une interface (40) propre à autoriser un équilibre osmotique avec le contenu de chaque goutte (20) à séparer ;
- l'interface (40) est formée par au moins une autre goutte (20) à séparer, avantageusement par une pluralité de gouttes (20) à séparer placées au contact de chaque goutte (20) à séparer ;
- flux osmotique entre les gouttes (20A) d'un premier groupe de gouttes et l'interface (40) pour modifier le volume de chaque goutte (20A) du premier groupe de gouttes ;
l'étape de flux osmotique provoquant une variation significative de la densité de chaque goutte (20A) du premier groupe de gouttes ;
- séparation des gouttes (20A, 20B) suivant leur densité ou suivant une combinaison de la densité et du volume pour isoler les gouttes (20A) du premier groupe de gouttes des gouttes (20B) d'un deuxième groupe de gouttes.

2. Procédé selon la revendication 1, **caractérisé en ce qu'**au moins une autre goutte (20) à séparer, avantageusement une pluralité d'autres gouttes (20) à séparer présentent une variation de densité inverse par rapport à la variation de densité des gouttes (20A) du premier groupe de gouttes lors de l'échange osmotique.

3. Procédé de sélection d'au moins un premier groupe de gouttes (20A) parmi une pluralité de gouttes (20) présentes dans une phase continue (24), les deux fluides formant respectivement la phase continue et les gouttes étant immiscibles, le procédé comprenant les étapes suivantes :
- mise en contact de gouttes (20) à séparer avec une interface (40) propre à autoriser un équilibre osmotique avec le contenu de chaque goutte (20) à séparer ;
l'interface (40) étant délimitée par une surface plane (60) d'une membrane semi-perméable (58) susceptible d'autoriser un équilibre osmotique avec le contenu de chaque goutte (20) à séparer, les gouttes à séparer (20) étant placées sur la surface plane (60) et disposées au contact de la membrane (58), une surface opposée (62) de la membrane (58) étant mise en contact avec un fluide de même nature et de même potentiel chimique que le liquide initialement contenu dans les gouttes (20);
- flux osmotique entre les gouttes (20A) d'un premier groupe de gouttes et l'interface (40) pour modifier le volume de chaque goutte (20A) du premier groupe de gouttes ;
l'étape de flux osmotique provoquant une variation significative de la densité de chaque goutte (20A) du premier groupe de gouttes ;
- séparation des gouttes (20A, 20B) suivant leur densité ou suivant une combinaison de la densité et du volume pour isoler les gouttes (20A) du premier groupe de gouttes des gouttes (20B) d'un deuxième groupe de gouttes.

4. Procédé selon l'une quelconque des revendications précédentes, **caractérisé en ce que** la variation significative de densité entre la densité initiale de chaque goutte (20A) du premier groupe de gouttes avant échange osmotique et la densité finale de chaque goutte (20A) du premier groupe de gouttes après échange osmotique est supérieure à 12%, notamment supérieure à 25%.

5. Procédé selon l'une quelconque des revendications précédentes, **caractérisé en ce que** le nombre de gouttes (20) à séparer est supérieur à 10⁶, le rapport du nombre de gouttes (20A) du premier groupe de gouttes au nombre total de gouttes (20) à séparer étant inférieur à 1/10000.

6. Procédé selon l'une quelconque des revendications précédentes, **caractérisé en ce que** l'étape de séparation comporte la disposition des gouttes (20A, 20B) dans un milieu liquide de densité différente d'au moins une parmi la densité de chacune des gouttes (20A) du premier groupe de gouttes et parmi la densité de chacune des gouttes (20B) du deuxième groupe de gouttes, le procédé comprenant la formation avantageusement spontanée d'au moins deux couches (50, 52) de gouttes, une première couche (50) comprenant majoritairement des gouttes (20A) du premier groupe de gouttes, la deuxième couche (52) comprenant majoritairement des gouttes (20B) du deuxième groupe de gouttes.

7. Procédé selon la revendication 6, **caractérisé en ce que** l'étape de séparation comporte la centrifugation du milieu contenant les gouttes (20A, 20B).

8. Procédé selon l'une quelconque des revendications 6 ou 7, **caractérisé en ce que** le procédé comprend l'ajout d'un additif dans le milieu liquide contenant les gouttes (20A, 20B) pour faire varier la densité du milieu liquide, l'ajout de l'additif étant réalisé avant l'étape de séparation, en particulier avant l'étape de flux osmotique, ou lors de l'étape de séparation.

9. Procédé selon l'une quelconque des revendications 6 à 8, **caractérisé en ce que** l'étape de séparation comporte la variation de la température du milieu liquide pour faire varier de manière différentielle la densité du milieu liquide, la densité des gouttes (20A) du premier groupe de gouttes et la densité des gouttes (20B) du deuxième groupe de gouttes.

10. Procédé selon l'une quelconque des revendications précédentes, **caractérisé en ce qu'**il comporte avant l'étape de mise en contact, la réaction sélective dans certaines gouttes (20) à séparer d'au moins un composé contenu dans une pluralité de gouttes (20) à séparer, pour former les gouttes (20A) du premier groupe de gouttes.

11. Procédé selon la revendication 10, **caractérisé en ce que** la réaction est une réaction chimique ou une réaction biologique.

12. Procédé selon la revendication 11, **caractérisé en ce que** les gouttes à séparer (20) contiennent initialement un même agent réactionnel, chaque goutte à séparer (20) contenant un réactif distinct, l'agent réactionnel étant susceptible de faire réagir le réactif.

13. Procédé selon la revendication 11, **caractérisé en ce que** les gouttes à séparer (20) contiennent initialement un même réactif, chaque goutte à séparer contenant un agent réactionnel distinct, l'agent réactionnel étant susceptible de faire réagir le réactif.

14. Procédé selon l'une quelconque des revendications précédentes, **caractérisé en ce que** les gouttes à séparer comprennent un composant non soluble dans la phase continue représentant initialement plus de 5% en masse de la masse de chaque goutte à séparer, le composant non soluble étant avantageusement un polymère de masse molaire supérieur à 10000 g/mol.

## Patentansprüche

1. Verfahren zum Auswählen mindestens einer ersten Gruppe von Tropfen (20A) aus einer Mehrzahl von in einer kontinuierlichen Phase (24) vorhandenen Tropfen (20), wobei die beiden Flüssigkeiten die kontinuierliche Phase bilden und die Tropfen nicht mischbar sind, wobei das Verfahren die folgenden Schritte aufweist:
- In-Kontakt-Bringen der zu trennenden Tropfen (20) mit einer Grenzfläche (40), die dazu geeignet ist, ein osmotisches Gleichgewicht mit dem Inhalt jedes der zu trennenden Tropfen (20) herzustellen;
- wobei die Grenzfläche (40) mittels mindestens eines anderen zu trennenden Tropfens (20) gebildet wird, vorzugsweise mittels einer Mehrzahl von zu trennenden Tropfen (20), die mit jedem der zur trennenden Tropfen (20) in Kontakt gebracht werden;
- osmotisches Austauschen zwischen den Tropfen (20A) einer ersten Gruppe von Tropfen und der Grenzfläche (40) zum Verändern des Volumens jedes Tropfens (20A) der ersten Gruppe von Tropfen;
wobei der Schritt des osmotischen Austauschens eine signifikante Änderung der Dichte jedes Tropfens (20A) der ersten Gruppe von Tropfen bewirkt;
- Auftrennen der Tropfen (20A, 20B) entsprechend ihrer Dichte oder entsprechend einer Kombination der Dichte und des Volumens zum Abtrennen der Tropfen (20A) der ersten Gruppe von Tropfen von den Tropfen (20B) einer zweiten Gruppe von Tropfen.

2. Verfahren gemäß Anspruch 1, **dadurch gekennzeichnet, dass** mindestens ein weiterer zu trennender Tropfen (20), vorzugsweise eine Mehrzahl von weiteren zu trennenden Tropfen (20), bei dem osmotischen Austausch eine Änderung der Dichte aufweisen, die umgekehrt zu der Änderung der Dichte der Tropfen (20A) der ersten Gruppe von Tropfen ist.

3. Verfahren zum Auswählen mindestens einer ersten Gruppe von Tropfen (20A) aus einer Mehrzahl von in einer kontinuierlichen Phase (24) vorhandenen Tropfen (20), wobei die beiden Flüssigkeiten die kontinuierliche Phase bilden und die Tropfen nicht mischbar sind, wobei das Verfahren die folgenden Schritte aufweist:
- In-Kontakt-Bringen von zu trennenden Tropfen (20) mit einer Grenzfläche (40), die dazu geeignet ist, ein osmotisches Gleichgewicht mit dem Inhalt jedes zu trennenden Tropfens (20) herzustellen;
wobei die Grenzfläche (40) mittels einer ebenen Oberfläche (60) einer semipermeablen Membran (58), die dazu geeignet ist, ein osmotisches Gleichgewicht mit dem Inhalt jedes zu trennenden Tropfens (20) herzustellen, begrenzt ist, wobei die zu trennenden Tropfen (20) auf die ebene Oberfläche (60) gebracht werden und in Kontakt mit der Membran (58) angeordnet werden, wobei eine entgegengesetzte Oberfläche (62) der Membran (58) mit einer Flüssigkeit, die eine gleichartige Beschaffenheit und ein gleiches chemisches Potential hat, wie die ursprünglich in den Tropfen (20) enthaltene Flüssigkeit, in Kontakt gebracht wird;
- osmotisches Austauschen zwischen den Tropfen (20A) einer ersten Gruppe von Tropfen und der Grenzfläche (40) zum Verändern des Volumens jedes Tropfens (20A) der ersten Gruppe von Tropfen;
wobei der Schritt des osmotischen Austauschens eine signifikante Änderung der Dichte jedes Tropfens (20A) der ersten Gruppe von Tropfen bewirkt;
- Auftrennen der Tropfen (20A, 20B) entsprechend ihrer Dichte oder entsprechend einer Kombination der Dichte und des Volumens zum Abtrennen der Tropfen (20A) der ersten Gruppe von Tropfen von den Tropfen (20B) einer zweiten Gruppe von Tropfen.

4. Verfahren gemäß einem der vorangehenden Ansprüche,
**dadurch gekennzeichnet, dass** die signifikante Änderung der Dichte zwischen der anfänglichen Dichte jedes Tropfens (20A) der ersten Gruppe von Tropfen vor dem osmotischen Austausch und der endgültigen Dichte jedes Tropfens (20A) der ersten Gruppe von Tropfen nach dem osmotischen Austausch größer als 12 % ist, insbesondere größer als 25 %.

5. Verfahren gemäß einem der vorangehenden Ansprüche,
**dadurch gekennzeichnet, dass** die Anzahl der zu trennenden Tropfen (20) größer als 10⁶ ist, wobei das Verhältnis der Anzahl von Tropfen (20A) der ersten Gruppe von Tropfen zu der Gesamtzahl von zu trennenden Tropfen (20) kleiner als 1/10000 ist.

6. Verfahren gemäß einem der vorangehenden Ansprüche,
**dadurch gekennzeichnet, dass** der Schritt des Auftrennens das Einbringen der Tropfen (20A, 20B) in ein flüssiges Medium einer Dichte, die verschieden ist von mindestens einer aus der Dichte jedes der Tropfen (20A) der ersten Gruppe von Tropfen und aus der Dichte jedes der Tropfen (20B) der zweiten Gruppe von Tropfen, aufweist, wobei das Verfahren das vorzugsweise spontane Bilden von mindestens zwei Schichten (50, 52) von Tropfen aufweist, wobei eine erste Schicht (50) mehrheitlich die Tropfen (20A) der ersten Gruppe von Tropfen aufweist, wobei die zweite Schicht (52) mehrheitlich die Tropfen (20B) der zweiten Gruppe von Tropfen aufweist.

7. Verfahren gemäß Anspruch 6, **dadurch gekennzeichnet, dass** der Schritt des Auftrennens das Zentrifugieren des flüssigen Mediums, das die Tropfen (20A, 20B) enthält, aufweist.

8. Verfahren gemäß Anspruch 6 oder 7, **dadurch gekennzeichnet, dass** das Verfahren das Hinzufügen eines Zusatzstoffes in das flüssige Medium, das die Tropfen (20A, 20B) aufweist, zum Verändern der Dichte des flüssigen Mediums aufweist, wobei das Hinzufügen des Zusatzstoffes vor dem Schritt des Auftrennens, insbesondere vor dem Schritt des osmotischen Austauschens, oder während des Schrittes des Auftrennens vorgenommen wird.

9. Verfahren gemäß einem der Ansprüche 6 bis 8, **dadurch gekennzeichnet, dass** der Schritt des Auftrennens das Verändern der Temperatur des flüssigen Mediums zum Verändern, auf unterschiedliche Weise, der Dichte des flüssigen Mediums, der Dichte der Tropfen (20A) der ersten Gruppe von Tropfen und der Dichte der Tropfen (20B) der zweiten Gruppe von Tropfen aufweist.

10. Verfahren gemäß einem der vorangehenden Ansprüche,
**dadurch gekennzeichnet, dass** es, vor dem Schritt des In-Kontakt-Bringens, in bestimmten zu trennenden Tropfen (20) das selektive Reagieren mindestens einer in der Mehrzahl von zu trennenden Tropfen (20) enthaltenen Verbindung zum Bilden der Tropfen (20A) der ersten Gruppe von Tropfen aufweist.

11. Verfahren gemäß Anspruch 10, **dadurch gekennzeichnet, dass** die Reaktion eine chemische Reaktion oder eine biologische Reaktion ist.

12. Verfahren gemäß Anspruch 11, **dadurch gekennzeichnet, dass** die zu trennenden Tropfen (20) anfänglich ein gleiches Reaktionsmittel aufweisen, wobei jeder zu trennende Tropfen (20) ein unterschiedliches Reagenz aufweist, wobei das Reaktionsmittel dazu geeignet ist, das Reagenz reagieren zu lassen.

13. Verfahren gemäß Anspruch 11, **dadurch gekennzeichnet, dass** die zu trennenden Tropfen (20) anfänglich ein gleiches Reagenz aufweisen, wobei jeder zu trennende Tropfen ein unterschiedliches Reaktionsmittel aufweist, wobei das Reaktionsmittel dazu geeignet ist, das Reagenz reagieren zu lassen.

14. Verfahren gemäß einem der vorangehenden Ansprüche,
**dadurch gekennzeichnet, dass** die zu trennenden Tropfen einen nicht-löslichen Bestandteil in der kontinuierlichen Phase aufweisen, der anfänglich mehr als 5 Masse-% der Masse jedes zu trennenden Tropfens darstellt, wobei der nicht-lösliche Bestandteil vorzugsweise ein Polymer einer Molmasse von mehr als 10000 g/mol ist.

## Claims

1. A method for selecting at least one first group of drops (20A) from among a plurality of drops (20) present in a continuous phase (24), the two fluids respectively forming the continuous phase and the drops are immiscible, the method comprising the following steps:
- bringing drops (20) to be separated into contact with an interface (40) suitable for allowing an osmotic equilibrium between the content of each drop (20) to be separated;
- the interface (40) being formed by at least one other drop (20) to be separated, advantageously by multiple drops (20) to be separated placed in contact with each drop (20) to be separated;
- osmotic flow between the drops (20A) of a first group of drops and the interface (40) in order to modify the volume of each drop (20A) of the first group of drops;
the osmotic flow step causing a significant variation in the density of each drop (20A) of the first group of drops;
- separating the drops (20A, 20B) according to the density thereof or according to a combination of the density and the volume to isolate the drops (20A) of the first group of drops from the drops (20B) of a second group of drops.

2. The method according to claim 1, **characterized in that** at least one other drop (20) to be separated, advantageously multiple other drops (20) to be separated, have an inverse density variation relative to the density variation of the drops of the first group of drops (20A) during the osmotic exchange.

3. A method for selecting at least one first group of drops (20A) from among a plurality of drops (20) present in a continuous phase (24), the two fluids respectively forming the continuous phase and the drops are immiscible, the method comprising the following steps:
- bringing drops (20) to be separated into contact with an interface (40) suitable for allowing an osmotic equilibrium between the content of each drop (20) to be separated;
- the interface (40) being delimited by a planar surface (60) of a semi-permeable membrane (58) suitable for allowing an osmotic equilibrium between the content of each drop (20) to be separated, the drops (20) to be separated being placed on the plane surface (60), and being positioned in contact with the membrane (58);an opposite surface (62) of the membrane (58) being brought into contact with a fluid of a same nature and same chemical potential as the liquid initially contained in the drops (20);
- osmotic flow between the drops (20A) of a first group of drops and the interface (40) in order to modify the volume of each drop (20A) of the first group of drops,
the osmotic flow step causing a significant variation in the density of each drop (20A) of the first group of drops;
- separating the drops (20A, 20B) according to the density thereof or according to a combination of the density and the volume to isolate the drops (20A) of the first group of drops from the drops (20B) of a second group of drops.

4. The method according to any one of the preceding claims, **characterized in that** the significant density variation between the initial density of each drop (20A) of the first group of drops before osmotic exchange and the final density of each drop (20A) of the first group of drops after osmotic exchange is greater than 12%, in particular greater than 25%.

5. The method according to any one of the preceding claims, **characterized in that** the number of drops (20) to be separated is greater than 10⁶, the ratio of the number of drops (20A) of the first group of drops to the total number of drops (20) to be separated being lower than 1/10,000.

6. The method according to any one of the preceding claims, **characterized in that** the separating step includes placing the drops (20A, 20B) in a liquid medium with a density that is different from at least one among the density of each of the drops (20A) of the first group of drops and among the density of each of the drops (20B) of the second group of drops, the method comprising the advantageously spontaneous formation of at least two layers (50, 52) of drops, a first layer (50) comprising primarily drops (20A) from the first group of drops, the second layer (52) comprising primarily drops (20B) from the second group of drops.

7. The method according to claim 6, **characterized in that** the separating step includes the centrifugation of the medium containing the drops (20A, 20B).

8. The method according to any one of claims 6 or 7, **characterized in that** the method comprises the addition of an additive in the liquid medium containing the drops (20A, 20B) to vary the density of the liquid medium, the addition of the additive being done before the separating step, in particular before the osmotic flow step, or during the separating step.

9. The method according to any one of claims 6 to 8, **characterized in that** the separating step includes varying the temperature of the liquid medium so as to differentially vary the density of the liquid medium, the density of the drops (20A) of the first group of drops and the density of the drops (20B) of the second group of drops.

10. The method according to any one of the preceding claims, **characterized in that** before the step for bringing into contact, it includes the selective reaction in certain drops (20) to be separated of at least one component contained in a plurality of drops (20) to be separated, to form the drops (20A) of the first group of drops

11. The method according to claim 10, **characterized in that** the reaction is a chemical reaction or a biological reaction.

12. The method according to claim 11, **characterized in that** the drops to be separated (20) initially contain a same reactive agent, each drop (20) to be separated containing a distinct reagent, the reactive agent being suitable for causing the reagent to react.

13. The method according to claim 11, **characterized in that** the drops to be separated (20) initially contain a same reagent, each drop to be separated containing a distinct reactive agent, the reactive agent being capable of causing the reagent to react.

14. The method according to any one of the preceding claims, **characterized in that** the drops to be separated contain a component that is not soluble in the continuous phase initially representing more than 5 wt% of the mass of each drop to be separated, the non-soluble component advantageously being a polymer with a molar mass greater than 10,000 g/mol.
